# EUROPEAN PATENT SPECIFICATION

(11) **EP 3 815 716 B1**
(45) Date of publication and mention of the grant of the patent: **06.09.2023**
(21) Application number: 19206690.0
(22) Date of filing: 31.10.2019
(51) Int. Cl.: A61L 9/12

(54) **DEVICE FOR DISPENSING MULTIPLE FRAGRANCES OR AROMAS, AND OPERATING METHOD THEREOF**
VORRICHTUNG ZUR AUSGABE VON MEHREREN DUFTSTOFFEN ODER AROMEN UND BETRIEBSVERFAHREN DAFÜR
DISPOSITIF DE DISTRIBUTION DE SENTEURS OU D'ARÔMES MULTIPLES ET SON PROCÉDÉ DE FONCTIONNEMENT

(43) Date of publication of application: 05.05.2021
(73) Proprietor: Panapanas Inc. Ltd, N-3406 Nassau (BS); Galvão, Cláudia, 05440-050 São Paulo (BR)
(72) Inventor: Galvão, Cláudia, 05440-050 São Paulo (BR)
(74) Representative: Patentree

(56) References cited:
- EP-A1- 3 815 717
- WO-A1-00/12143
- WO-A1-2020/094577
- WO-A1-2020/190933
- US-A1- 2003 107 139
- US-A1- 2004 241 053
- US-B1- 6 231 032

## Description

### TECHNICAL FIELD

The present disclosure relates to the field of dispersing and testing fragrances or aromas, and refers to a device, which acts like an electromechanical catalogue, capable of containing a plurality of fragrances or aromas.

### BACKGROUND

The current state of the art anticipates some patent documents related to testing devices for fragrances and aromas, such as US5167877A entitled "Air purifier with perfume dispensing control" - used in the air purifying sector, the equipment described releases different perfumes, through the passage of air coming from a pump, for the cartridge of previously-selected perfume and consequent outlet of the perfume into the environment. It uses a solenoid air pump and carousel driven by electromagnet.

The apparatus above does not have a friendly interface with the user due to the lack of electronics, which would enable commands by means of applications and control of the intensity and scope of perfume emission.

Mechanically, the prior art presents constructive complexity, as it is made up of many components, besides requiring a considerably powerful solenoid pump, in view of the fact that the air is pumped into a liquid medium (perfume) to produce bubbles and thus bring the aroma to the outlet orifice. It has been proven that systems using bubbling to draw the aroma do not provide a good olfactory experience.

Still mechanically speaking, the prior art has a rotary table driven by electromagnet, whereas the return to the starting point occurs by means of springs. The mechanism is imprecise and is subject to wear and tear, for example, in the springs which will increase the imprecision or will mal function when their elasticity is lost.

Lastly, the constructive complexity and the excess of components may cause defects due to handling during demonstration, besides making its size rather voluminous.

US 2004/0241053 A1, entitled "Apparatus for dispensing volatile material into the environment" - used in the air aromatization sector. The equipment consists of a set of elements, with perfume disposed on a rotary disk, such that when positioning the chosen element an air flow generated by a blower traverses the element, loading the perfume into the environment. The document cites a perfume heating element to facilitate volatilization. The disk positioning system is electronic and controlled by sensors, motors and electronic circuits.

It is known that heating perfumes distorts them as the more volatile notes are released first. Another drawback is that there is an excess of mechanical components such as: gearings, shafts, couplings, which result in an apparatus of considerable volume, besides hindering assembly and making the operation liable to defects or breakage of components.

WO02/09776 A2, entitled "System and methods for dispensing scents into the environment, and for providing scent-containing articles of manufacture" - the equipment described pertains to the sector of dispensing multiple volatile products into the environment, including perfumes. A disk receives various cartridges containing the volatile substances. An electronic system that can be commanded by a computer, or directly on the equipment, positions the chosen cartridge and by means of heating and passage of air coming from fan, the substance is released into the environment.

As in the previous document, the use of heating to release the fragrances, mischaracterizes the substances due to the premature evaporation of more volatile notes.

Document WO2000012143A1, for which the preamble of claims 1 and 15 relates, relates to an odour dispensing device comprising a housing and a disc shaped dispensing cartridge adapted to move around its rotation axis and having a plurality of discrete radically arranged compartments, each compartment containing an odorant on an odorant carrier, said housing containing means for positioning the cartridge, means for producing an airstream to a preselected compartment in response to a signal emanating from a computer control module, a microprocessor, an optical system or a timing mechanism and means for temporarily subjecting the odour carrier within the compartment to the airstream so that an odour is discharged from the cartridge and entrained in the airstream, and to an odour dispensing cartridge

These facts are disclosed in order to illustrate the technical problem addressed by the present disclosure.

### GENERAL DESCRIPTION

A device for dispensing multiple fragrances or aromas, and operating method thereof, according to the present invention, is characterised by the features recited in the characterising portion of claims 1 and 15, respectively.

The present disclosure relates to the field of dispersing and testing fragrances or aromas, and refers to a device, which acts like an electromechanical catalogue, capable of containing a plurality of fragrances or aromas, in particular from one to 100 (a hundred) types of a plurality of fragrances or aromas. When connected to a wired or wireless computerized system, which will preferably command it by means of an application, this device is capable of finding the selected fragrance in a carousel, and then carrying out a controlled burst of the air aromatized by the fragrance contained in the cartridge duly positioned in the carousel per se, providing the user a reliable olfactory experience, since it uses the concept of "dry scent", since in the exhalation system there is no heating, which would mischaracterize the fragrance by the evaporation of the more volatile notes. If the user wishes to try another fragrance or aroma, he/she simply chooses it in the application, and the apparatus will select the fragrance chosen.

The electromechanical device for dispersing multiple fragrances or aromas invented is a compact electronic catalogue capable of offering the user "dry" olfactory testing of different fragrances or aromas. The device in question is formed by a box with a movement mechanism that comprises a carousel, which may accommodate up to one hundred cartridges containing fragrances or aromas, duly contained in an absorbent element, in particular an absorbent cylindrical element. On being triggered via application, the device means that the selected cartridge, housed in said carousel, turns, thanks to an electric motor with gearbox, up to the connector nozzle. By means of a mechanical actuator, driven by electric motor, gearing and rack, the cartridge is nudged inside the connector nozzle, at which time it receives an air flow coming from a compressor, which when traversing a flexible hose, forces the elastomeric projections that act as a valve of a path, thus occupying the cartridge and, spilling out only the gaseous portion of the fragrance to the upper outlet orifice of the cartridge per se, thus enabling a reliable olfactory testing. Therefore, when the cartridge is displaced forward, its top, which incorporates the valve of a path, displaces the pivoting handle which in turn releases a fragrance or aroma to the outlet orifice. Once testing has finished, the cartridge returns to the starting position. At this moment, the pivoting handle also returns closing the outlet orifice of the box and of the cartridge, which prevents the fragrances / aromas from mixing. Each fragrance or aroma is contained in a cartridge (refill), and these cartridges disposed in a carousel, and in particular each cartridge may offer over 30 bursts (testings). The cartridges may be easily replaced, as they are encased in the carousel and accessible by way of a recharge window outside the casing.

It is an objective of the present disclosure to propose an electromechanical device that acts as an electronic catalogue for demonstrating multiple fragrances or aromas, wherein the chosen cartridge is mechanically moved to the emission position, and ventilated so that the air draws the scented vapours to the outlet orifice;

It is an objective of the present disclosure to propose an electromechanical device, ecologically correct and sustainable, since it prevents on a monthly basis the consumption of tons of paper used in the traditional direct sales catalogues;

In an embodiment, an aspect of the disclosure is to propose an electromechanical device for demonstrating multiple fragrances or aromas, which uses the concept of "dry scent", being activated by air ventilation, means the fragrances / aromas do not lose their original characteristics, since they did not undergo the action of elements liable to alter them, as occurs in the electro-thermal activation devices. It is known that heating the fragrance / aroma mischaracterizes its scent, since in the first heating, more volatile compounds (evaporating before others) are lost. A fragrance is composed of a great number of chemical substances, each having a different boiling point (evaporation). Normally, the scent exhaled by pulverization or evaporation is not absorbed appropriately and/or with precision by the human brain already on the fourth testing, according to studies carried out in the perfumery segment.

It is an objective of the present disclosure to propose an electromechanical device for demonstrating multiple fragrances or aromas, whose concept of "dry scent" enables the testing, with precision and real aroma, various fragrances. In this embodiment of the invention it is possible to test twenty fragrances with distinction of the notes of the perfumes;

It is an objective of the present disclosure to propose an electromechanical device for demonstrating multiple fragrances or aromas, which will be used as a digital catalogue to present olfactory tests.

It is an objective of the present disclosure to propose an electromechanical device for demonstrating multiple fragrances and aromas, capable of offering a multiple and controlled olfactory testing for the user.

It is an objective of the present disclosure to propose an electromechanical device for demonstrating multiple fragrances and aromas, wherein the user using the apparatus connected to an electronic device with its application, may replace the kits of glass flasks, olfactory strips or, catalogues of samples and the equipment that use heating in the release of the scent and mischaracterize the fragrances.

It is an objective of the present disclosure to propose an electromechanical device for demonstrating multiple fragrances and aromas, the working principle of which saves on fragrances and aromas, since the device does not pulverize, does not bubble, does not nebulize, nor does it heat the fragrances and aromas.

It is an objective of the present disclosure to propose an electromechanical device for demonstrating multiple fragrances and aromas, which solves the drawbacks of other equipment, which contaminate the surfaces of the very emitting device, leaving it impregnated with the residual scent, adversely affecting the olfactory experience by the mixture of the scents. This device presents the advantage of the internal air flow of the system is impregnated only inside the cartridge, and released directly to the outlet orifice, eliminating the possibility of the mixture of the aromas or fragrances.

It is an objective of the present disclosure to propose an electromechanical device for demonstrating multiple fragrances and aromas, which solves the drawbacks of other equipment, which store the scents when not in use, since it may optionally have an autoclean system which, after the use, conducts all the remaining scent outwardly of the device.

It is an objective of the present disclosure to propose an electromechanical device for demonstrating multiple fragrances and aromas, capable of generating greater savings on fragrances or aromas, since there is no wastage with nebulization, bubbling or pulverization that require greater volumes. As already described, there are savings on glass flasks used for demonstrating perfumes, as well paper strips (olfactory strips).

It is an objective of the present disclosure to propose an electromechanical device for demonstrating multiple fragrances and aromas, compact in size, similar to a tablet, which facilitates transport and handling, and with capacity for up to 100 (one hundred) types of fragrances / aromas.

It is an objective of the present disclosure to propose an electromechanical device for demonstrating multiple fragrances and aromas, capable of generating a large number of bursts, for demonstrating and appreciating multiple fragrances and aromas, and which may replace the traditional glass flasks of samples of fragrances/aromas. This is possible due to the reduced volume inside the cartridges, associated to the ventilation of the concentrates of fragrances / aromas. The result is a satisfactory olfactory experience, in time, purity, intensity, reproducibility and durability of the cartridges, when compared to the current showcases.

It is an objective of the present disclosure to propose an electromechanical device for demonstrating multiple fragrances and aromas that offers a satisfactory experience at a competitive cost, when compared with the current systems of samples in flasks and showcases.

It is an objective of the present disclosure to propose an electromechanical device for demonstrating multiple fragrances and aromas, capable of being operated or controlled by various electronic interface means. Besides providing an application or program for making the selection and emission of the aromas, the system may also be driven by buttons in its box.

It is an objective of the present disclosure to propose an electromechanical device for demonstrating multiple fragrances and aromas, having optimal cost/benefit ratio, since it presents a reduced number of components, with consequent drop in defects and facilitation of the manufacturing process, and quality control.

It is disclosed a device for dispensing multiple fragrances or aromas comprising:
a plurality of interchangeable individual cartridges each comprising an absorbent element containing a fragrance or aroma and an outlet orifice for emission of the fragrance or aroma;
a carousel comprising said plurality of cartridges encased in said carousel;
a drawing cane for: pushing a selected cartridge from the carousel for connecting with a connector nozzle; and retracting the selected cartridge back to the carousel;
an air compressor and a hose connecting the air compressor and the connector nozzle, for inflating air through the hose and the connector nozzle to enter the pushed cartridge for emission of the fragrance or aroma through the outlet orifice;
a mechanical actuator for driving the drawing cane;
an electric motor for rotating the carousel;
an electronic circuit connected to said air compressor, actuator, motor, and arranged for rotating the carousel until the selected cartridge is aligned with the connector nozzle, pushing the selected cartridge from the carousel to connect with the connector nozzle, and inflating air through the hose and the connector nozzle to enter the pushed cartridge for emission of the fragrance or aroma.

It is also disclosed a method for operating a device for dispensing multiple fragrances or aromas, said device comprising:
a plurality of interchangeable individual cartridges each comprising an absorbent element containing a fragrance or aroma and an outlet orifice for emission of the fragrance or aroma;
a carousel comprising said plurality of cartridges encased in said carousel;
a drawing cane for: pushing a selected cartridge from the carousel for connecting with a connector nozzle; and retracting the selected cartridge back to the carousel;
an air compressor and a hose connecting the air compressor and the connector nozzle, for inflating air through the hose and the connector nozzle to enter the pushed cartridge for emission of the fragrance or aroma through the outlet orifice;
a mechanical actuator for driving the drawing cane;
an electric motor for rotating the carousel;
an electronic circuit connected to said air compressor, actuator, motor;
the method comprising the steps of:
   rotating the carousel until the selected cartridge is aligned with the connector nozzle;
   pushing the selected cartridge from the carousel to connect with the connector nozzle;
   inflating air through the hose and the connector nozzle to enter the pushed cartridge for emission of the fragrance or aroma.

In an embodiment, the absorbent element is cylindrical and impregnated with the fragrance or aroma.

An embodiment comprises a circular rack incorporated to the carousel for being driven by the electric motor.

An embodiment comprises a reducer and a gearing mounted on a shaft of said reducer for driving the circular rack.

In an embodiment, the hose is flexible.

An embodiment comprises a battery provided with external connectors for feeding the electronic circuit.

An embodiment comprises central bearings for supporting the carousel and a circular path for the carousel to glide in.

An embodiment comprises a calibration sensor for detecting a starting position of the carousel in respect of a reference cartridge aligned with the connector nozzle.

It is also disclosed a non-transitory storage media including program instructions for implementing a method for operating a device for dispensing multiple fragrances or aromas, the program instructions including instructions executable by a data processor to carry the method of any of the described embodiments.

### BRIEF DESCRIPTION OF THE DRAWINGS

The following figures provide preferred embodiments for illustrating the disclosure and should not be seen as limiting the scope of invention.

The disclosure will now be described in further embodiments, and for better understanding, references will be made to the accompanying drawings, representing the following:

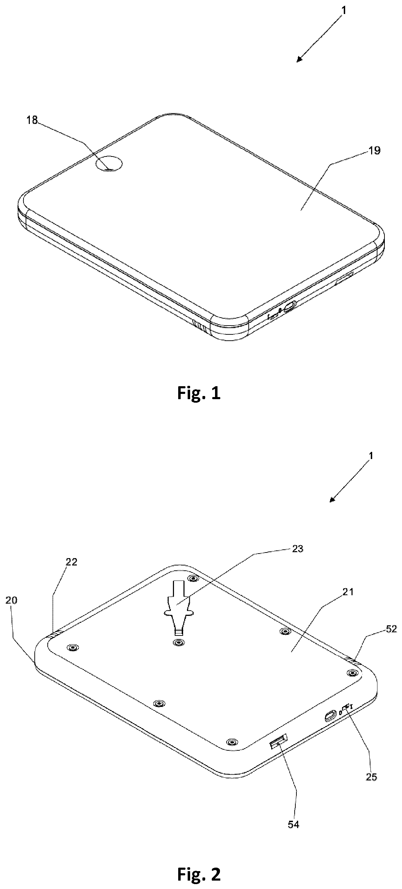

**Figure 1****:** Schematic representation of an embodiment of a perspective view of the wireless electromechanical device for demonstrating multiple fragrances or aromas.
**Figure 2****:** Schematic representation of an embodiment of a reverse perspective view of the wireless electromechanical device for demonstrating multiple fragrances or aromas.

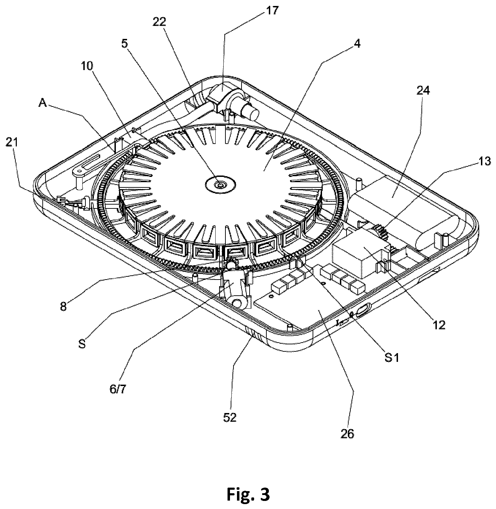

**Figure 3****:** Schematic representation of an embodiment of a perspective view of the wireless electromechanical device for demonstrating multiple fragrances or aromas, without the top.

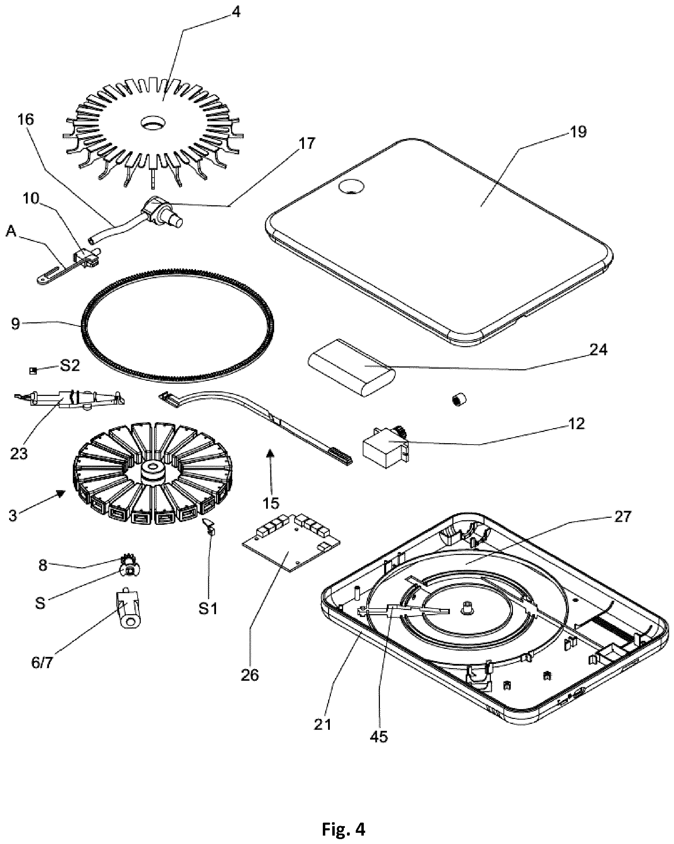

**Figure 4****:** Schematic representation of an embodiment of an exploded perspective view of the wireless electromechanical device for demonstrating multiple fragrances or aromas.

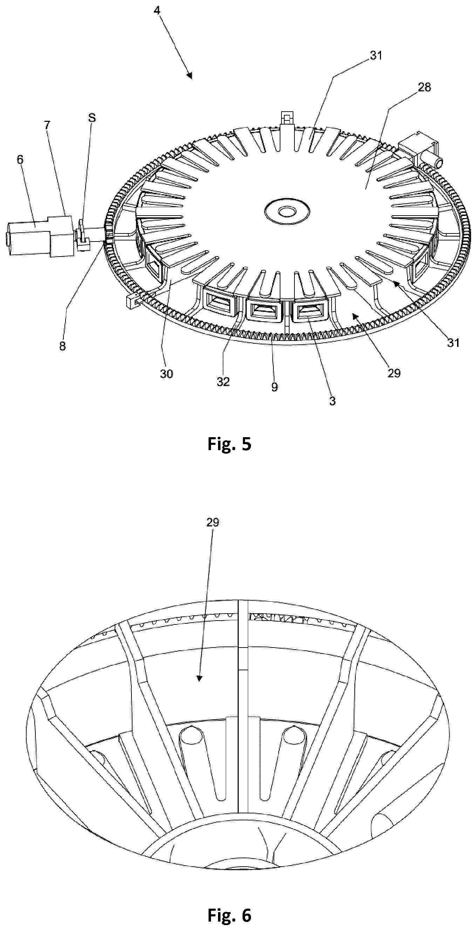

**Figure 5****:** Schematic representation of an embodiment of a perspective view of the carousel and turning mechanism wireless electromechanical device for demonstrating multiple fragrances or aromas.
**Figure 6****:** Schematic representation of an embodiment of a reverse perspective view of the carousel and turning mechanism wireless electromechanical device for demonstrating multiple fragrances or aromas.

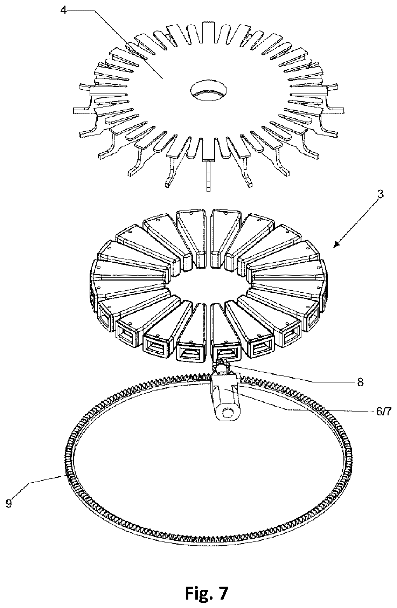

**Figure 7****:** Schematic representation of an embodiment of an exploded perspective view of the set of the carousel and turning mechanism wireless electromechanical device for demonstrating multiple fragrances or aromas.

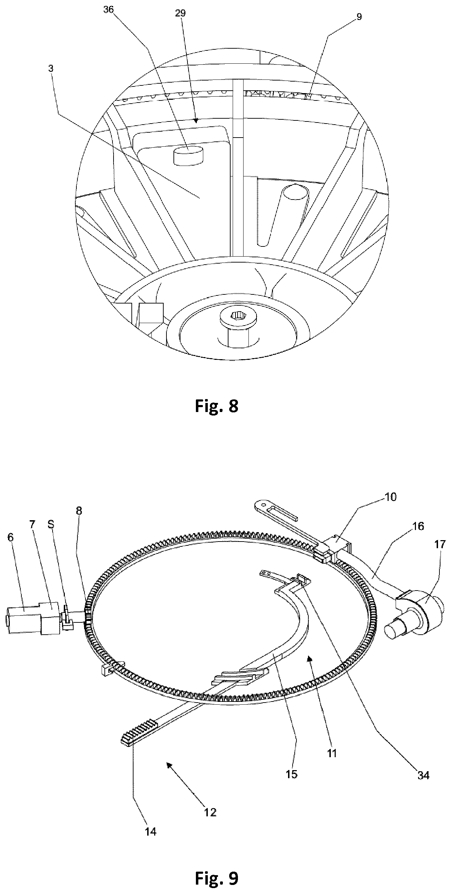

**Figure 8****:** Schematic representation of an embodiment of a perspective view of the wireless electromechanical device for demonstrating multiple fragrances or aromas, showing the cartridge mounted on the carousel.
**Figure 9****:** Schematic representation of an embodiment of a perspective view of the drive mechanism of the cartridge of the wireless electromechanical device for demonstrating multiple fragrances or aromas, shown without the carousel.

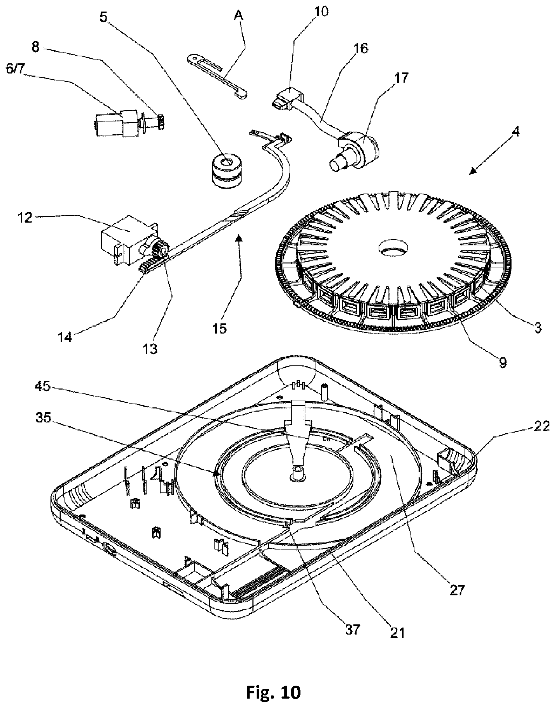

**Figure 10****:** Schematic representation of an embodiment of an exploded perspective view of the forward mechanism of the cartridge of the wireless electromechanical device for demonstrating multiple fragrances or aromas, shown with the carousel and ventilation system.

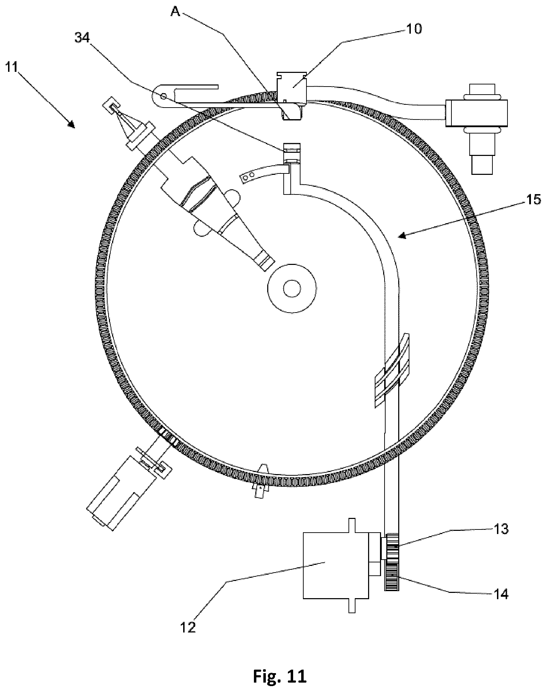

**Figure 11****:** Schematic representation of an embodiment of a top view of the forward mechanism of the cartridge of the wireless electromechanical device for demonstrating multiple fragrances or aromas.

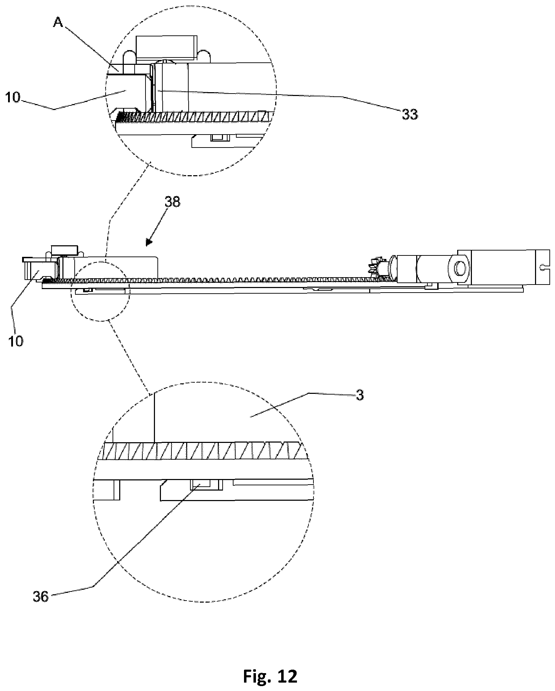

**Figure 12****:** Schematic representation of an embodiment of a side view of the forward mechanism of the cartridge of the wireless electromechanical device for demonstrating multiple fragrances or aromas, with the cartridge recoiled and detail of the lower setting of the cartridge to the arm.

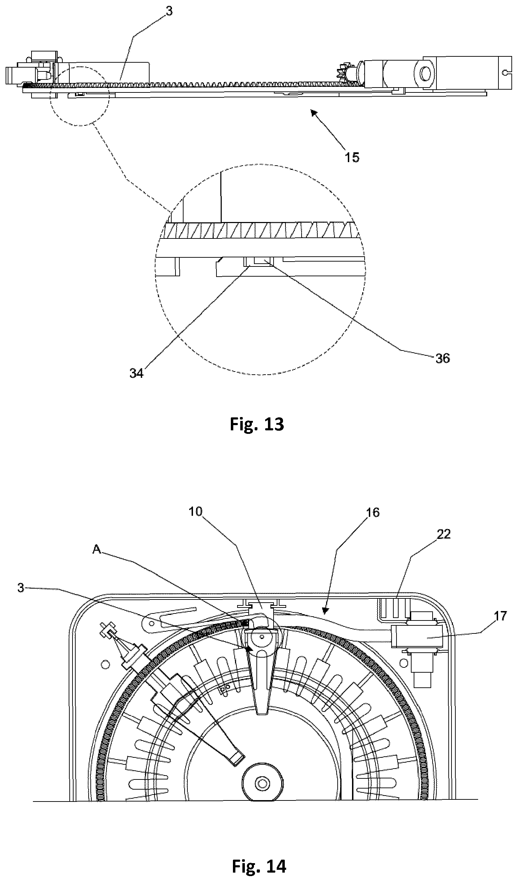

**Figure 13****:** Schematic representation of an embodiment of a side view of the forward mechanism of the cartridge of the wireless electromechanical device for demonstrating multiple fragrances or aromas, with the cartridge advanced.
**Figure 14****:** Schematic representation of an embodiment of a perspective view of the flexible ventilation hose of the cartridge of the wireless electromechanical device for demonstrating multiple fragrances or aromas, with detail of the air intake orifice.

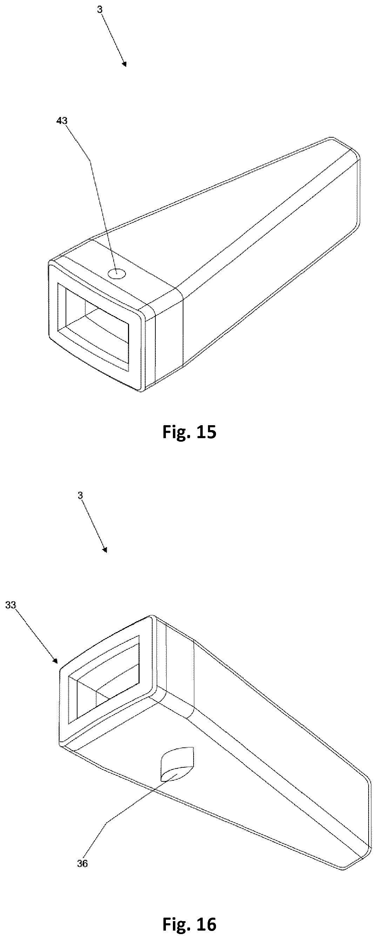

**Figure 15****:** Schematic representation of an embodiment of a perspective view of the cartridge of the forward mechanism of the cartridge of the wireless electromechanical device for demonstrating multiple fragrances or aromas.
**Figure 16****:** Schematic representation of an embodiment of a reverse perspective view of the cartridge of the forward mechanism of the cartridge of the wireless electromechanical device for demonstrating multiple fragrances or aromas.

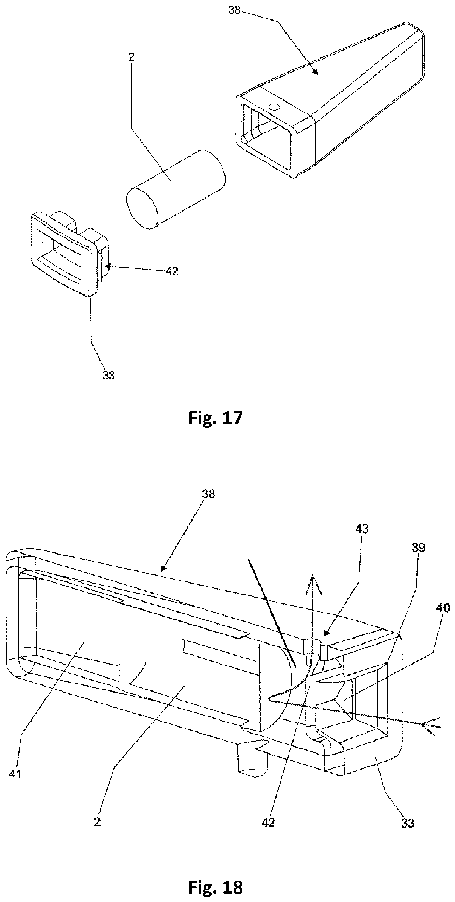

**Figure 17****:** Schematic representation of an embodiment of an exploded perspective view of the cartridge of the wireless electromechanical device for demonstrating multiple fragrances or aromas, shown the air flow.
**Figure 18****:** Schematic representation of an embodiment of a cutaway view of the cartridge of the wireless electromechanical device for demonstrating multiple fragrances or aromas, shown the air flow, with detail inferior of the passage of air of the header.

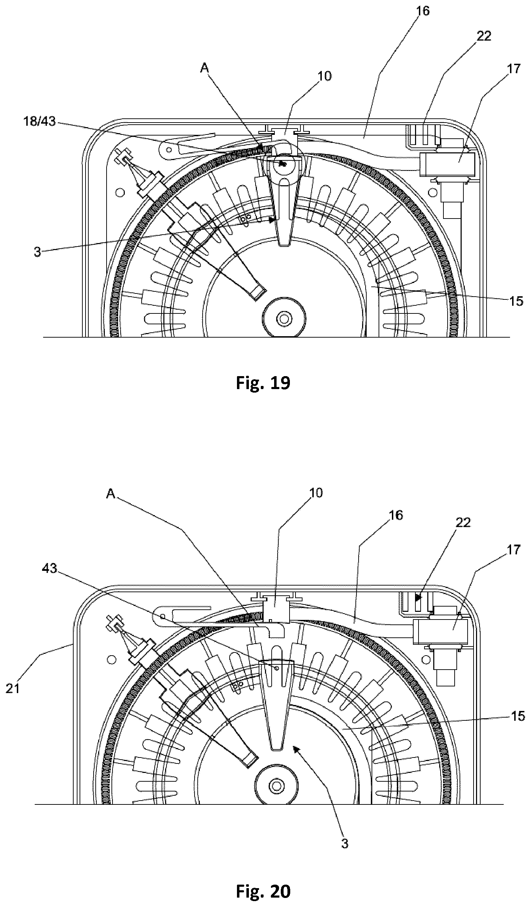

**Figure 19****:** Schematic representation of an embodiment of an upper schematic view illustrating the cartridge of the wireless electromechanical device for demonstrating multiple fragrances or aromas, in a forward position to release the fragrance.
**Figure 20****:** Schematic representation of an embodiment of an upper schematic view illustrating the cartridge of the wireless electromechanical device for demonstrating multiple fragrances or aromas, in a recoiled position to retain the fragrance.

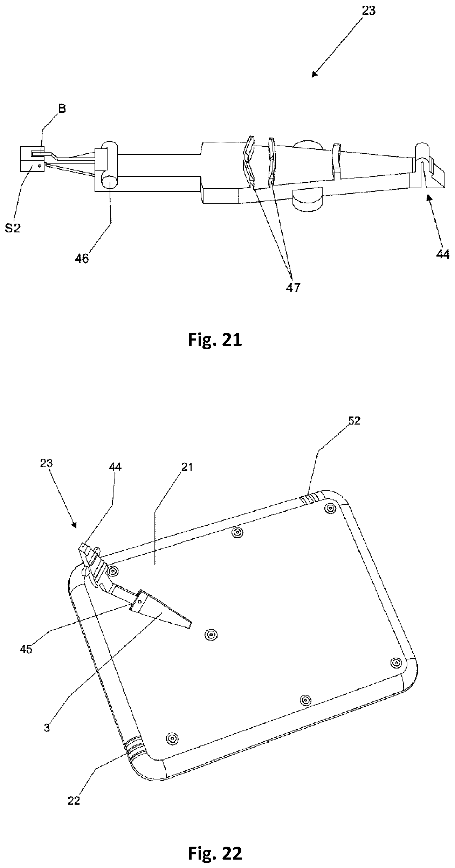

**Figure 21****:** Schematic representation of an embodiment of a perspective view of the wireless electromechanical device for demonstrating multiple fragrances or aromas, showing the top for changing the cartridge.
**Figure 22****:** Schematic representation of an embodiment of a perspective view of the wireless electromechanical device for demonstrating multiple fragrances or aromas, showing the top for changing the cartridge installed and in open position.

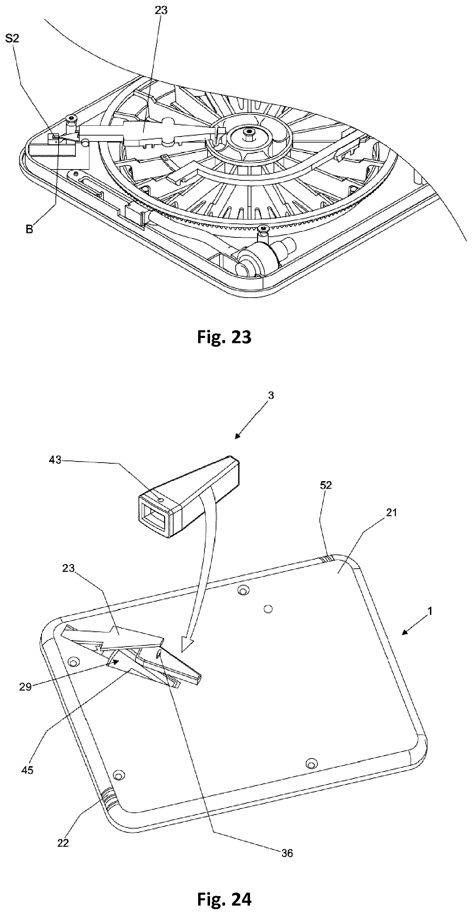

**Figure 23****:** Schematic representation of an embodiment of a perspective view of the wireless electromechanical device for demonstrating multiple fragrances or aromas, showing the top sensor for changing the open cartridge.
**Figure 24****:** Schematic representation of an embodiment of a perspective view of the wireless electromechanical device for demonstrating multiple fragrances or aromas, showing the change of the cartridge.

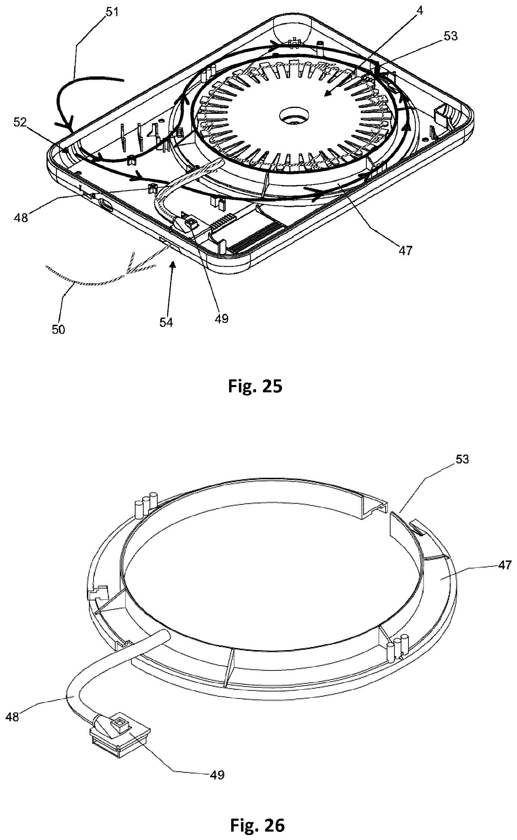

**Figure 25****:** Schematic representation of an embodiment of a schematic perspective view of the wireless electromechanical device for demonstrating multiple fragrances or aromas, showing optional autoclean mechanism.
**Figure 26****:** Schematic representation of an embodiment of a perspective view of the wireless electromechanical device for demonstrating multiple fragrances or aromas, showing optional autoclean mechanism components.

### DETAILED DESCRIPTION

The present disclosure relates to a device 1, which acts like an electronic catalogue for the olfactory testing of different fragrances or aromas, contained in an absorbent cylindrical element 2 inserted into individual cartridges **3,** which are interchangeable and encased in a carousel **4,** supported on central bearings **5** and radially moved by means of an electric motor **6** with reducer **7** and gearing **8** which acts on a circular rack **9.** Upon receipt of a command, via application, the carousel **4** turns, positioning the cartridge **3,** corresponding to the fragrance or aroma chosen, in front of the connector nozzle **10,** thanks to a positioning sensor **S,** which counts the turns of the shaft of the electric motor **6.** Next, a mechanical actuator **11** formed by electric motor **12,** gearing **13** which operates on a straight rack **14,** which in turn drives a drawing cane **15,** which pushes, and then retracts the cartridge, **3** inside the connector nozzle **10,** and jointly with the air flow coming from a flexible hose **16,** from where the air inflated by a compressor **17** enters said cartridge **3,** forces the emission of the scent through the outlet orifice **18** of the top **19** of the box **20,** thus providing the user a reliable olfactory testing.

More particularly, electromechanical device **1** claimed is formed by a bipartite box **20,** having a bottom **21** endowed with tears **22** at the side end, for air intake in the compressor **17** and port **23** for recharging the cartridges **3,** and a top **19** with outlet orifice **18** of the fragrances. The bottom **21** contains a rechargeable battery **24,** which is provided with energy by means of external connectors **25,** with the objective of powering an electronic circuit **26** responsible for the interface application/electromechanical device **1** and for the correct location of the cartridge **3,** performed by the sensor and positioning **S,** containing a fragrance chosen by the user, in relation to the connector nozzle **10.** Lastly, the box **20** also comprises an electric motor **6** for driving the turning mechanism of the carousel **4,** which receives the cartridges **3,** besides an electric motor **12** for driving the mechanical actuator **11,** as well as the compressor **17** and an easy-to-assemble flexible hose **16** which receives the air coming from the compressor **17,** leading it to the connector nozzle **10.**

The electromechanical device **1,** which, as already commented upon, is nothing more than an electronic catalogue with different fragrances, contained in an absorbent cylindrical element **2** inserted into the individual cartridges **3,** operates as follows.

As soon as the electromechanical device **1** is switched on, a calibration sensor **S1** places the reference cartridge **3** in line with the connector nozzle **10.** Next, the cartridge **3,** with the fragrance chosen by the user in the application, begins turning for alignment in the emission position, that is, in front of the connector nozzle **10,** using the positioning sensor **S** installed in the shaft of the motor **6 /** reducer **7** showing the number of turns of this shaft with the position of the cartridges **3.** The carousel **4** is turned by means of an electric motor **6,** reducer **7** in whose there is coupled a gearing **8** which acts on a circular rack **9** solidary to the carousel **4,** which turns, as already commented upon, until the selected cartridge **3** aligns with the connector nozzle **10.**

The carousel **4** is formed by a single circular part, solidary to the rack **9,** supported on central bearings **5,** which enables the movement thereof with minimum attrition on a circular path **27,** marked at the bottom **21** of the box **20** of the electromechanical device **1.** The circular rack **9,** jointly with the electric motor **6,** reducer **7** and gearing **8** assure the circular gliding of said carousel **4** and, consequently, of the cartridges **3** encased therein.

The carousel **4** is circular in shape with a straight portion **28,** projected vertically, forming a series of perimeter housings **29** substantially triangular in shape, formed by side walls **30** and upper prolongation **31,** said straight portion **28** being projected vertically linked to the rack **9** by means of appendices **32.** Accordingly, the perimeter housings **29** are suitable for adequately receiving the cartridges **3,** which are compatible in shape.

Therefore, the perimeter housings **29** and the type of encasement of the cartridge **3** promote a longitudinal freedom of movement in relation to the carousel **4,** since the cartridge **3** presents a certain mobility between the side walls **30** and upper prolongation **31.** The entrance to the perimeter housing **29** faces the elastomeric header **33** of the cartridges **3.**

As soon as it is aligned with the connector nozzle **10,** the cartridge **3** is moved by the mechanical actuator **11.** The cartridge **3** is advanced by the electric motor **12** which when switched on drives a gearing **13** that activates a straight rack **14,** which is displaced forwards or backwards, a drawing cane **15** linked to the cartridge **3** in the direction of the connector nozzle **10.** Accordingly, the end of the drawing cane **15** has a recess **34,** which is an integral part of the track **35** of the circular path **27,** which constitutes a stopper in the lower projection **36** of the cartridge **3.**

Therefore, once aligned by the positioning sensor **S,** the carousel **4** with the cartridge **3** chosen by the user remains aligned with the connector nozzle **10.** The mechanical actuator **11,** by means of a drawing cane **15,** substantially C-shaped, is displaced in a guide groove **37,** at the bottom **21** of the box **20,** in the direction of the connector nozzle **10.** At its distal end, the drawing cane **15** presents the recess **34,** which constitutes a stopper in the lower projection **36** of the cartridge **3,** such that the movement of both parts is brought together, that is, if the drawing cane **15** advances towards the connector nozzle **10** the cartridge **3** also advances and vice-versa. It is worth recalling, as already commented upon, that the cartridge **3** is fastened, but with freedom of longitudinal movement, on the carousel **4.** When the cartridges **3** are mounted in the reel **9,** the lower projections **36** constitute a stopper on the track **35** moulded at the bottom **21** of the box **20,** which attributes stability and uniform gliding.

After the cartridge **3** has advanced, the forced air flow is made present by the compressor **17.** The emission of the aroma begins when the air flow taken in by the compressor **17** in the tears **22** passes through the flexible hose **16,** enters the connector nozzle **10** and traverses the inside of the cartridge **3,** with the chosen fragrance chosen, already positioned.

The cartridge **3** is mounted in a prismatic triangle shape casing **38,** endowed with an elastomeric header **33** which constitutes an inlet valve **39** of a path, represented by converging flexible pallets **40.** Internally, the cartridge **3** receives an absorbent cylindrical element **2,** which impregnated with a fragrance or aroma, configures a long-lasting scent chamber **41.** In the elastomeric header **33,** which fits perfectly into the casing **38,** in addition to the inlet valve **39** of a path, which is actuated and/or opened when the cartridge **3** enters into the connector nozzle **10,** there are also side passages **42,** which direct the fragrance or aroma to the upper outlet orifice **43** of the cartridge **3,** through which the scent is exhaled.

The present disclosure with the cartridge **3,** duly allocated, the mechanical actuator **11** pushes it towards the connector nozzle **10** to the emission position. In a previous step, the air flow takes the flexible hose **16** and the converging flexible pallets **40** of the inlet valve **39** of a path are retracted, giving passage to the air towards the scent chamber **41,** already saturated by the fragrance or aroma contained in the absorbent cylindrical element **2.** Lastly, the air impregnated with a fragrance or aroma can only escape through the side passages **42** of the elastomeric header **33,** which end on the upper outlet orifice **43,** through which the fragrance is exhaled. In this position, the cartridge **3** advanced, by way of the elastomeric header **33** force the displacement of the covering pivoting handle **A,** of the upper outlet orifice **43** of the cartridge **3** and of the outlet orifice **18** of the top **19,** in the sense of releasing both orifices, and with the elastomeric header **33** already inserted into the connector nozzle **10,** the aroma or fragrance is released into the environment. As soon as the cartridge **3** is recoiled to manoeuvring or turning position, the covering pivoting handle **A** once again seals the outlet orifice **18** of the top **19,** the inlet valve **39** of a path also returns and its converging flexible pallets **40** once again seals thus preventing fragrance leakage. When recoiled, the upper outlet orifice **43** is closed by interference with a straight portion **28** of the carousel **4.**

To make the change, the user must choose the fragrance or aroma to be changed in the application, so that the carousel **4** positions the refill under the recharge port **23** of the cartridges **3,** located at the bottom **21** of the box **20** of the electromechanical device **1.** The recharge port **23** of the cartridges **3** presents a triangular end where there is a fold **44** which enables by means of a click in an opening **45,** having compatible size, located at the bottom **21** of the box **20** where it is pivoted by shaft **46** injected into the part itself. Still on the inner face of the recharge port **23** there are two parallel walls **47** that give continuity to the track **35.** Lastly, at the opposite end, a bung **B,** which acts in balance in relation to the shaft **46** constitutes a stopper on the recharge port **23** sensor **S2** of the cartridges **3** open, which prevents the carousel **4** from moving in this situation, which would lead to the overlapping of the cartridges **3.** In this context, the change is facilitated because the cartridges **3** remain freely positioned inside the perimeter housings **29,** it being suffice to open the recharge port **23,** remove the empty cartridge **3,** and then insert the refill cartridge **3** in the cavity of the housing **29** delimited by the side walls **30** and upper prolongation **31.**

Optionally, the electromechanical device **1** may incorporate an autoclean mechanism, which comprises a ring **47,** encircling the carousel **4,** which has the function of containing the fragrance or aroma in that region. The ring **47** is interconnected by means of hose **48** to an exhaust **49** which, when activated, conducts the air saturated **50** with the aromas or fragrances, from inside the carousel **4,** out of the electromechanical device **1.** The autoclean is activated whenever the electromechanical device **1** is switched off, or when the user deems necessary. Automatically, the exhaust **49** is activated and forces the entry of clean air **51** from the environment through the opening **52,** which enters the inner region of the carousel **4** by a second opening **53,** mixing the saturated air **50** following the path already mentioned until it is expelled through the window **54.** Autoclean is useful so that the electromechanical device **1,** for example, does not impregnate the bag of the demonstrators after testing.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

## Claims

1. Device (1) for dispensing multiple fragrances or aromas comprising:
a plurality of interchangeable individual cartridges (3) each comprising an absorbent element (2) containing a fragrance or aroma and an outlet orifice (43) for emission of the fragrance or aroma;
a carousel (4) comprising said plurality of cartridges (3) encased in said carousel (4);
a drawing cane (15);
a mechanical actuator (11) for driving the drawing cane (15);
an electric motor (6) for rotating the carousel (4);
**characterised in that**
the drawing cane (15) is arranged for: pushing a selected cartridge (3) from the carousel (4) for connecting with a connector nozzle (10); and retracting the selected cartridge (3) back
to the carousel (4);
wherein the device further comprises:
an air compressor (17) and a hose (16) connecting the air compressor (17) and the connector nozzle (10), for inflating air through the hose (16) and the connector nozzle (10) to enter the pushed cartridge (3) for emission of the fragrance or aroma through the outlet orifice (43);
an electronic circuit (26) connected to said air compressor, actuator (11), motor (6), and arranged for rotating the carousel until the selected cartridge (3) is aligned with the connector nozzle (10), pushing the selected cartridge (3) from the carousel (4) to connect with the connector nozzle (10), and inflating air through the hose (16) and the connector nozzle (10) to enter the pushed cartridge (3) for emission of the fragrance or aroma.

2. Device (1) according to any of the previous claims comprising a bipartite box (20) comprising a box orifice (18) for emission of the fragrance or aroma, located such that the box orifice (18) is aligned with the outlet orifice (43) of the pushed cartridge (3) for emission of the fragrance or aroma through both orifices (43, 18).

3. Device (1) according to any of the previous claims comprising a positioning sensor (S) connected to said electronic circuit, for detecting the position of the selected cartridge (3) in relation to the connector nozzle (10).

4. Device (1) according to the previous claim wherein the positioning sensor (S) is arranged to count the number of turns of the electric motor (6).

5. Device (1) according to any of the previous claims wherein the carousel (4) comprises a flat portion (28) from which a plurality of perimeter housings (29) project, wherein each perimeter housing (29) is a triangularly shaped projection for receiving a cartridge (3).

6. Device (1) according to any of the previous claims wherein the carousel (4) comprises a plurality of perimeter housings (29), each for receiving a cartridge (3) and having a prolongation (31) arranged for closing the outlet orifice (43) of the cartridge (3) when the cartridge is retracted in the carousel (4).

7. Device (1) according to any of the previous claims, wherein the cartridges (3) comprise a projection (36) and the drawing cane (15) comprises a recess (34), the device further comprising a guide groove (37) arranged to guide the drawing cane (15) for pushing and retracting the selected cartridge (3) by engaging the recess (34) with the projection (36).

8. Device (1) according to any of the previous claims wherein the cartridge (3) comprises a prismatic triangular shape casing (38) and an elastomeric header (33) arranged as an entrance valve (3), forming a chamber for enclosing the absorbent element (2) and the respective fragrance or aroma.

9. Device (1) according to any the previous claim wherein the elastomeric header (33) comprises converging flexible pallets (40) arranged to open when the pushed cartridge (3) connects with the connector nozzle (10) and to close when the pushed cartridge (3) retracts from with the connector nozzle (10), in particular the elastomeric header (33) further comprising side passages (42) for directing the fragrance or aroma to the outlet orifice (43) of the cartridge (3).

10. Device (1) according to any of the previous claims comprising a covering pivoting handle (A), wherein the elastomeric header (33) of the pushed cartridge (3) forces the displacement of said covering pivoting handle (A) to open, if existing, the box orifice (18), and to release the outlet orifice (43) for emission of the fragrance or aroma.

11. Device (1) according to the previous claim wherein the covering pivoting handle (A) is arranged to recoil, when the pushed cartridge (3) is retracted, to seal the outlet orifice (43) and, if existing, the box orifice (18).

12. Device (1) according to any of the previous claims comprising a bipartite box (20) which comprises a recharge opening (45) and a recharge port (23) for covering said recharge opening (45), both arranged to enable the exchange of one of the cartridges (3) of the carousel (4).

13. Device (1) according to the previous claim wherein the device comprises a circular path (27) for the carousel (4) to glide in and a recharge port (23) which is pivotable around a shaft (46), the recharge port (23) comprising two inwardly facing parallel walls (47) for providing track (35) continuity to the circular path (27).

14. Device (1) according to any of the previous claims further comprising an autoclean mechanism which comprises a ring (47) encircling the carousel (4) for containing fragrances or aromas in a region defined by said ring, a hose (48) connected to said ring, and an exhaust (49) connected to said hose (48), arranged such that when the exhaust (49) is activated, air (50) with the aromas or fragrances is lead from inside the ring (47) to the outside of the device (1).

15. Method for operating a device (1) for dispensing multiple fragrances or aromas, said device comprising:
a plurality of interchangeable individual cartridges (3) each comprising an absorbent element (2) containing a fragrance or aroma and an outlet orifice (43) for emission of the fragrance or aroma;
a carousel (4) comprising said plurality of cartridges (3) encased in said carousel (4);
a drawing cane (15);
a mechanical actuator (11) for driving the drawing cane (15);
an electric motor (6) for rotating the carousel;
**characterised in that**
the drawing cane (15) is arranged for: pushing a selected cartridge (3) from the carousel (4) for connecting with a connector nozzle (10); and retracting the selected cartridge (3) back to the carousel (4);
wherein the device further comprises:
an air compressor (17) and a hose (16) connecting the air compressor (17) and the connector nozzle (10), for inflating air through the hose (16) and the connector nozzle (10) to enter the pushed cartridge (3) for emission of the fragrance or aroma through the outlet orifice (43);
an electronic circuit (26) connected to said air compressor, actuator (11), motor (6);
the method comprising the steps of:
rotating the carousel until the selected cartridge (3) is aligned with the connector nozzle (10);
pushing the selected cartridge (3) from the carousel (4) to connect with the connector nozzle (10);
inflating air through the hose (16) and the connector nozzle (10) to enter the pushed cartridge (3) for emission of the fragrance or aroma.

## Patentansprüche

1. Vorrichtung (1) zur Ausgabe mehrerer Duftstoffe oder Aromen, umfassend:
eine Vielzahl austauschbarer einzelner Kartuschen (3), jeweils umfassend ein absorbierendes Element (2), das einen Duftstoff oder ein Aroma enthält, und eine Austrittsöffnung (43) zur Ausgabe des Duftstoffes oder Aromas;
ein Karussell (4), umfassend die genannte Vielzahl der Kartuschen (3), die in das genannte Karussell (4) eingelassen sind;
einen Zieharm(15);
einen mechanischen Aktor (11) als Antrieb für den Zieharm (15);
einen Elektromotor (6) zum Drehen des Karussells (4);
**dadurch gekennzeichnet, dass**
der Zieharm (15) so angeordnet ist, dass er eine ausgewählte Kartusche (3) aus dem Karussell (4) zieht; diese mit einem Verbindungsstutzen (10) verbindet; und
die ausgewählte Kartusche (3) in das Karussell (4) zurückzieht;
wobei die Vorrichtung ferner umfasst:
einen Luftkompressor (17) und einen Schlauch (16), der den Luftkompressor (17) mit dem Verbindungsstutzen (10) verbindet, um Luft durch den Schlauch (16) und den Verbindungsstutzen (10) in die herausgezogene Kartusche (3) zu pumpen, um den Duftstoff oder das Aroma durch die Austrittsöffnung (43) abzugeben;
einen elektronischen Schaltkreis (26), der mit dem genannten Luftkompressor, dem Aktor (11) und dem Motor (6) verbunden und so angeordnet ist, dass er das Karussell solange dreht, bis die ausgewählte Kartusche (3) mit dem Verbindungsstutzen (10) ausgerichtet ist und die ausgewählte Kartusche (3) von dem Karussell (4) schiebt, um sie mit dem Verbindungsstutzen (10) zu verbinden, und um Luft durch den Schlauch (16) und den Verbindungsstutzen (10) zu pumpen, um die herausgeschobene Kartusche (3) für die Ausgabe des Duftstoffs oder des Aromas einzuführen.

2. Vorrichtung (1) nach dem vorangehenden Anspruch, umfassend ein zweiteiliges Gehäuse (20), das eine Gehäuseöffnung (18) zur Ausgabe des Duftstoffes oder Aromas aufweist, die so angeordnet ist, dass die Gehäuseöffnung (18) mit der Austrittsöffnung (43) der herausgeschobenen Kartusche (3) zur Ausgabe des Duftstoffes oder Aromas durch beide Öffnungen (43, 18) ausgerichtet ist.

3. Vorrichtung (1) nach einem der vorangehenden Ansprüche, umfassend einen Positionierungssensor (S), der mit dem genannten elektronischen Schaltkreis verbunden ist, um die Position der ausgewählten Kartusche (3) in Bezug zu dem Verbindungsstutzen (10) zu erfassen.

4. Vorrichtung (1) nach dem vorangehenden Anspruch, wobei der Positionierungssensor (S) so angeordnet ist, dass er die Anzahl der Umdrehungen des Elektromotors (6) zählt.

5. Vorrichtung (1) nach einem der vorangehenden Ansprüche, wobei das Karussell (4) einen flachen Abschnitt (28) aufweist, von dem eine Vielzahl von Aufnahmegehäusen (29) vorsteht, wobei jedes Aufnahmegehäuse (29) ein dreieckig geformter Vorsprung zur Aufnahme einer Kartusche (3) ist.

6. Vorrichtung (1) nach einem der vorangehenden Ansprüche, wobei das Karussell (4) eine Vielzahl von Aufnahmegehäusen (29) umfasst, die jeweils zur Aufnahme einer Kartusche (3) dienen und eine Verlängerung (31) aufweisen, die zum Verschließen der Austrittsöffnung (43) der Kartusche (3) angeordnet ist, wenn die Kartusche in das Karussell (4) zurückgezogen wird.

7. Vorrichtung (1) nach einem der vorangehenden Ansprüche, wobei die Kartusche (3) einen Vorsprung (36) aufweisen und der Zieharm (15) eine Aussparung (34) aufweist, wobei die Vorrichtung ferner eine Führungsnut (37) aufweist, die so angeordnet ist, dass sie den Zieharm (15) zum Schieben und Zurückziehen der ausgewählten Kartusche (3) durch das Ineinandergreifen der Aussparung (34) und des Vorsprungs (36) führt.

8. Vorrichtung (1) nach einem der vorangehenden Ansprüche, wobei die Kartusche (3) ein prismatisches, dreieckiges Gehäuse (38) und einen elastomeren Kopf (33) umfasst, der als ein Eingangsventil (3) angeordnet ist und eine Kammer zur Aufnahme des absorbierenden Elements (2) und des jeweiligen Duftstoffs oder Aromas bildet.

9. Vorrichtung (1) nach einem der vorangehenden Ansprüche, wobei der elastomere Kopf (33) konvergierende bewegliche Flächen (40) umfasst, die so angeordnet sind, dass sie sich öffnen, wenn die herausgeschobene Kartusche (3) mit dem Verbindungsstutzen (10) verbunden wird, und dass sie sich schließen, wenn die herausgeschobene Kartusche (3) von dem Verbindungsstutzen (10) zurückgezogen wird, wobei der elastomere Kopf (33) ferner insbesondere seitliche Durchgänge (42) umfasst, um den Duftstoff oder das Aroma zur Austrittsöffnung (43) der Kartusche (3) zu leiten.

10. Vorrichtung (1) nach einem der vorangehenden Ansprüche, die einen schwenkbaren Abdeckungsgriff (A) umfasst, wobei der elastomere Kopf (33) der herausgeschobenen Kartusche (3) durch das Verschieben des schwenkbaren Abdeckungsgriffs (A) dafür sorgt, dass sich die Gehäuseöffnung (18), falls vorhanden, öffnet und die Austrittsöffnung (43) für die Ausgabe des Duftstoffs oder Aromas freigibt.

11. Vorrichtung (1) nach dem vorangehenden Anspruch, wobei der schwenkbare Abdeckungsgriff (A) so angeordnet ist, dass er zurückspringt, wenn die herausgeschobene Kartusche (3) zurückgezogen wird, um die Austrittsöffnung (43) und, falls vorhanden, die Gehäuseöffnung (18) zu verschließen.

12. Vorrichtung (1) nach einem der vorangehenden Ansprüche, umfassend ein zweiteiliges Gehäuse (20), umfassend eine Nachladeöffnung (45) und eine Nachladebuchse (23) zum Abdecken der genannten Nachladeöffnung (45), die beide so angeordnet sind, dass sie den Austausch einer der Kartuschen (3) des Karussells (4) ermöglichen.

13. Vorrichtung (1) nach dem vorangehenden Anspruch, wobei die Vorrichtung eine kreisförmige Bahn (27) umfasst, auf der das Karussell (4) gleitet, und eine Nachladeöffnung (23) umfasst, die um eine Welle (46) schwenkbar ist, wobei die Nachladeöffnung (23) zwei nach innen weisende parallele Wände (47) umfasst, um der kreisförmigen Bahn (27) eine kontinuierliche Spur (35) zu verleihen.

14. Vorrichtung (1) nach einem der vorangehenden Ansprüche, ferner umfassend einen Selbstreinigungsmechanismus, umfassend einen Ring (47), der das Karussell (4) umgibt, um Duftstoffe oder Aromen in einem durch den genannten Ring definierten Bereich aufzunehmen, einen Schlauch (48), der mit dem genannten Ring verbunden ist, und einen Auslass (49), der mit dem genannten Schlauch (48) verbunden und so angeordnet ist, dass, wenn der Auslass (49) aktiviert wird, Luft (50) mit den Aromen oder Duftstoffen aus dem Inneren des Rings (47) zur Außenseite der Vorrichtung (1) geleitet wird.

15. Verfahren zum Betrieb einer Vorrichtung (1) zur Ausgabe mehrerer Duftstoffe oder Aromen, wobei die genannte Vorrichtung umfasst:
eine Vielzahl austauschbarer einzelner Kartuschen (3), jeweils umfassend ein absorbierendes Element (2), das einen Duftstoff oder ein Aroma enthält, und eine Austrittsöffnung (43) zur Ausgabe des Duftstoffes oder Aromas umfasst;
ein Karussell (4), umfassend die genannte Vielzahl der Kartuschen (3), die in das genannte Karussell (4) eingelassen sind;
einen Zieharm (15);
einen mechanischen Aktor (11) als Antrieb für den Zieharm (15);
einen Elektromotor (6) zum Drehen des Karussells (4);
**dadurch gekennzeichnet, dass**
der Zieharm (15) so angeordnet ist, dass er eine ausgewählte Kartusche (3) aus dem Karussell (4) zieht; diese mit einem Verbindungsstutzen (10) verbindet; und
die ausgewählte Kartusche (3) in das Karussell (4) zurückzieht;
wobei die Vorrichtung ferner umfasst:
einen Luftkompressor (17) und einen Schlauch (16), der den Luftkompressor (17) mit dem Verbindungsstutzen (10) verbindet, um Luft durch den Schlauch (16) und den Verbindungsstutzen (10) in die herausgezogene Kartusche (3) zu pumpen, um den Duftstoff oder das Aroma durch die Austrittsöffnung (43) abzugeben;
einen elektronischen Schaltkreis (26), der mit dem genannten Luftkompressor, dem Aktor (11) und dem Motor (6) verbunden ist;
wobei das Verfahren folgende Schritte umfasst:
Drehen des Karussells bis die ausgewählte Kartusche (3) mit dem Verbindungsstutzen (10) ausgerichtet ist;
Schieben der ausgewählten Kartusche (3) vom Karussell (4), um sie mit dem Verbindungsstutzen (10) zu verbinden;
Einpumpen von Luft durch den Schlauch (16) und den Verbindungsstutzen (10), damit die herausgeschobene Kartusche (3) den Duftstoff oder das Aroma ausgibt.

## Revendications

1. Dispositif (1) de distribution de senteurs ou d'arômes multiples comprenant :
une pluralité de cartouches individuelles interchangeables (3) chacune comprenant un élément absorbant (2) contenant une senteur ou un arôme et un orifice de sortie (43) pour émettre la senteur ou l'arôme ;
un carrousel (4) comprenant ladite pluralité de cartouches (3) encastrées dans ledit carrousel (4) ;
une canne d'étirage (15) ;
un actionneur mécanique (11) pour faire fonctionner la canne d'étirage (15) ;
un moteur électrique (6) pour faire tourner le carrousel (4) ;
**caractérisé en ce que**
la canne d'étirage (15) est arrangée pour : pousser une cartouche sélectionnée (3) hors du carrousel (4) pour la raccorder à une buse de raccord (10) ; et rétracter la cartouche sélectionnée (3) dans le carrousel (4) ;
dans lequel le dispositif comprend également :
un compresseur d'air (17) et un tuyau (16) raccordant le compresseur d'air (17) et la buse de raccord (10), pour envoyer de l'air à travers le tuyau (16) et la buse de raccord (10) pour qu'il entre dans la cartouche poussée (3) pour émettre la senteur ou l'arôme à travers l'orifice de sortie (43) ;
un circuit électronique (26) raccordé audit compresseur d'air, actionneur (11), moteur (6), et arrangé pour faire tourner le carrousel jusqu'à ce que la cartouche sélectionnée (3) soit alignée avec la buse de raccord (10), poussant la cartouche sélectionnée (3) hors du carrousel (4) pour la raccorder à la buse de raccord (10), et envoyer de l'air à travers le tuyau (16) et la buse de raccord (10) pour qu'il entre dans la cartouche poussée (3) pour émettre la senteur ou l'arôme.

2. Dispositif (1) selon la revendication précédente comprenant une boîte bipartite (20) comprenant un orifice de boîte (18) pour émettre la senteur ou l'arôme, située telle que l'orifice de boîte (18) soit aligné avec l'orifice de sortie (43) de la cartouche poussée (3) pour émettre la senteur ou l'arôme à travers les deux orifices (43, 18).

3. Dispositif (1) selon l'une quelconque des revendications précédentes, comprenant un capteur de positionnement (S) raccordé audit circuit électronique, pour détecter la position de la cartouche sélectionnée (3) par rapport à la buse de raccord (10).

4. Dispositif (1) selon la revendication précédente dans lequel le capteur de positionnement (S) est arrangé pour compter le nombre de rotations du moteur électrique (6).

5. Dispositif (1) selon l'une quelconque des revendications précédentes dans lequel le carrousel (4) comprend une portion plate (28) de laquelle sont projetés une pluralité de compartiments de périmètre (29), dans lequel chaque compartiment de périmètre (29) est une projection en forme de triangle pour recevoir une cartouche (3).

6. Dispositif (1) selon l'une quelconque des revendications précédentes dans lequel le carrousel (4) comprend une pluralité de compartiments de périmètre (29), pour recevoir chacun une cartouche (3) et ayant un prolongement (31) arrangé pour fermer l'orifice de sortie (43) de la cartouche (3) lorsque la cartouche est rétractée dans le carrousel (4).

7. Dispositif (1) selon l'une quelconque des revendications précédentes, dans lequel les cartouches (3) comprennent une projection (36) et la canne d'étirage (15) comprend un renfoncement (34), le dispositif comprenant également une rainure de guidage (37) arrangée pour guider la canne d'étirage (15) pour pousser et rétracter la cartouche sélectionnée (3) en mettant le renfoncement (34) en prise avec la projection (36).

8. Dispositif (1) selon l'une quelconque des revendications précédentes dans lequel la cartouche (3) comprend un boîtier en forme de triangle prismatique (38) et une pièce de tête élastomère (33) arrangée en tant que valve d'entrée (3), formant une chambre pour renfermer l'élément absorbant (2) et la senteur ou l'arôme respectif.

9. Dispositif (1) selon l'une quelconque des revendications précédentes dans lequel la pièce de tête élastomère (33) comprend des palettes flexibles convergentes (40) arrangées pour s'ouvrir lorsque la cartouche poussée (3) se raccorde à la buse de raccord (10) et pour se fermer lorsque la cartouche poussée (3) est rétractée de la buse de raccord (10), en particulier la pièce de tête élastomère (33) comprenant également des passages latéraux (42) pour guider la senteur ou l'arôme vers l'orifice de sortie (43) de la cartouche (3).

10. Dispositif (1) selon l'une quelconque des revendications précédentes comprenant une poignée pivotante couvrante (A), dans lequel la pièce de tête élastomère (33) de la cartouche poussée (3) force le déplacement de ladite poignée pivotante couvrante (A) pour ouvrir, s'il existe, l'orifice de boîte (18), et pour relâcher l'orifice de sortie (43) pour émettre la senteur ou l'arôme.

11. Dispositif (1) selon la revendication précédente dans lequel la poignée pivotante couvrante (A) est arrangée pour reculer, lorsque la cartouche poussée (3) est rétractée, pour sceller l'orifice de sortie (43) et, s'il existe, l'orifice de boîte (18).

12. Dispositif (1) selon l'une quelconque des revendications précédentes comprenant une boîte bipartite (20) qui comprend une ouverture de recharge (45) et un port de recharge (23) pour couvrir ladite ouverture de recharge (45), tous deux étant arrangés pour permettre l'échange de l'une des cartouches (3) du carrousel (4).

13. Dispositif (1) selon la revendication précédente dans lequel le dispositif comprend une trajectoire circulaire (27) sur laquelle le carrousel (4) peut glisser et un port de recharge (23) qui peut être pivoté autour d'un arbre (46), le port de recharge (23) comprenant deux parois parallèles tournées vers l'intérieur (47) pour permettre la continuité de circuit (35) de la trajectoire circulaire (27).

14. Dispositif (1) selon l'une quelconque des revendications précédentes comprenant également un mécanisme de nettoyage automatique qui comprend un anneau (47) encerclant le carrousel (4) pour contenir les senteurs ou arômes dans une zone définie par ledit anneau, un tuyau (48) raccordé audit anneau, et un échappement (49) raccordé audit tuyau (48), arrangé tel que lorsque l'échappement (49) est activé, l'air (50) contenant les arômes ou senteurs soit guidé de l'intérieur de l'anneau (47) vers l'extérieur du dispositif (1).

15. Procédé de fonctionnement d'un dispositif (1) de distribution de senteurs ou d'arômes multiples, ledit dispositif comprenant :
une pluralité de cartouches individuelles interchangeables (3) chacune comprenant un élément absorbant (2) contenant une senteur ou un arôme et un orifice de sortie (43) pour émettre la senteur ou l'arôme ;
un carrousel (4) comprenant ladite pluralité de cartouches (3) encastrées dans ledit carrousel (4) ;
une canne d'étirage (15) ;
un actionneur mécanique (11) pour faire fonctionner la canne d'étirage (15) ;
un moteur électrique (6) pour faire tourner le carrousel ;
**caractérisé en ce que**
la canne d'étirage (15) est arrangée pour : pousser une cartouche sélectionnée (3) hors du carrousel (4) pour la raccorder à une buse de raccord (10) ; et rétracter la cartouche séléctionnée (3) dans le carrousel (4) ;
dans lequel le dispositif comprend également :
un compresseur d'air (17) et un tuyau (16) raccordant le compresseur d'air (17) et la buse de raccord (10), pour envoyer de l'air à travers le tuyau (16) et la buse de raccord (10) pour qu'il entre dans la cartouche poussée (3) pour émettre la senteur ou l'arôme à travers l'orifice de sortie (43) ;
un circuit électronique (26) raccordé audit compresseur d'air, actionneur (11), moteur (6) ;
le procédé comprenant les étapes de :
faire tourner le carrousel jusqu'à ce que la cartouche sélectionnée (3) soit alignée avec la buse de raccord (10) ;
pousser la cartouche sélectionnée (3) hors du carrousel (4) pour la raccorder à la buse de raccord (10) ;
envoyer l'air à travers le tuyau (16) et la buse de raccord (10) pour qu'il entre dans la cartouche poussée (3) pour émettre la senteur ou l'arôme.
